# EUROPEAN PATENT APPLICATION

(11) **EP 2 990 070 A1**
(43) Date of publication of application: **02.03.2016**
(21) Application number: 13882695.3
(22) Date of filing: 26.04.2013
(51) Int. Cl.: A61M 25/14, A61B 18/12

(54) **ABLATION CATHETER**

(71) Applicant: Terumo Kabushiki Kaisha, Tokyo 151-0072 (JP); Shanghai Angiocare Medical Technology Co., Ltd., Shanghai 201201 (CN)
(72) Inventor: KOBAYASHI, Junichi, Ashigarakami-gun Kanagawa 259-0151 (JP)
(74) Representative: Casalonga
(86) International application number: PCT/JP2013/062359
(87) International publication number: WO 2014/174662

(57) **Abstract**

The invention provides an ablation catheter capable of improving operability by extremely reducing anisotropy of torque transmitting performance depending on rotational direction while maintaining flexibility.

An ablation catheter includes a shaft body (60) extending in an axial direction (X) with a spiral slit (61) formed at least partially thereto, and an electrode tip (31) arranged at the distal end part of the shaft body (60) which can be inserted into a living body to thermally influence biological tissue, wherein the shaft body (60) comprising a portion having different spiral inclination angles (α) which is an angle of the extending direction of the spiral of the slit (61) against the cross section orthogonal to the axial direction (X), while step parts (102) where the angle of the slit to the spiral inclination angle (α) changes locally are formed on one of the opposing surfaces (100) comprising the slit (61), and contacting parts (112) with which the step parts (102) contact when the shaft body (60) is twisted to the direction to unwind the spiral of the slit (61) are formed on the other opposing surface (110), while the length (L3) of the step parts (102) in the axial direction (X) is formed to be longer in the portion where the spiral inclination angle (α) is relatively large than in the portion where the spiral inclination angle is small.

## Description

### Technical Field

The present invention relates to an ablation catheter to be inserted in a biological lumen to cauterize biological tissue.

### Background Art

In recent years, more focus is being placed on the validity of renal sympathetic nerve ablation as a treatment for therapy resistant high blood pressure. It is said that an accentuation of renal sympathetic activity is related to onset and maintenance of high blood pressure, and effect to lower the blood pressure of patients with therapy resistant high blood pressure by ablating renal sympathetic nerve is anticipated.

In renal sympathetic nerve ablation, a catheter with ablation function is introduced to a renal artery transdermally to ablate a renal sympathetic nerve that runs surrounding the renal artery by the catheter from inside the renal artery to give thermal damage.

The ablation catheter is provided with a long shaft to be inserted into a blood vessel. For example, in patent literature 1, a shaft having a tubular body with a spiral slit formed by laser-cutting to provide bendable spring-like structure is shown.

### Citation List

### Patent Literature

Patent Literature 1: Official gazette of JP Patent Publication No. 2012-513873

### Summary of Invention

### Technical Problem

However, operability of the catheter is degraded when a spiral slit is formed to the tubular body that comprises the shaft of the catheter as the torque transmitting performance differs by rotational direction due to anisotropy of the structure. That is, by rotating the base part of the catheter to one direction, the torque acts in the direction to close the gap between the slit, whereas by rotating it to the opposite direction, the torque acts in the direction to open the gap between the slit, in other words in the direction to unwind the spiral. When the torque acts in the direction to close the gap between the slit, torque can be transmitted efficiently as it is not possible for the slit to close more than the width of the gap formed in between, however when the torque acts in the direction to open the gap between the slit, the gap between the slit tends to open more than necessary, which results in inefficient transmittal of torque and tends to degrade operability.

The present invention is made to resolve the problem mentioned above, and aims to provide an ablation catheter capable of improving operability by extremely reducing anisotropy of torque transmitting performance due to rotational direction while maintaining flexibility.

### Solution to Problem

To achieve the above mentioned object, the ablation catheter is comprised of a tubular body extending in an axial direction with a spiral slit formed at least partially, and a heating element provided at the distal end part of the tubular body which can be inserted into a living body to thermally influence the biological tissue, wherein the tubular body includes portions having different spiral inclination angle which is an angle of the extending direction of the spiral of the slit to the cross section orthogonal to the axial direction, protruding parts formed to protrude from one side of the paired opposing surfaces that comprise the slit, and recessed parts formed on the other side of the opposing surfaces to allow the protruding parts to enter therein, further the protruding parts are formed with step parts in the portion where the angle of the slit to the spiral inclination angle changes locally, and the recessed parts are formed with contacting parts against which the step parts come in contact with when the tubular body is twisted to the direction to unwind the spiral of the slit, and the length of the step parts in the axial direction is formed to be longer in the portion where the spiral inclination angle is relatively large than in the portion where it is small.

### Advantageous Effects of Invention

According to the ablation catheter configured as mentioned above, it is possible to optionally set the flexibility depending on the position to the axial direction to improve operability by forming portions with different spiral inclination angle of the slit, and prevent the gap between the slit to open when the tubular body is twisted to the direction to unwind the spiral by forming the step parts and contacting parts to contact each other when the tubular body is twisted to the direction to unwind the spiral. Further, as the length of the step parts is formed to be longer in the portion where the spiral inclination angle is relatively large than in the portion where it is small, the length of the step parts is formed longer at the portion where the latching by the step parts are easily disengaged due to the larger spiral inclination angle of the slit to make it difficult to disengage from the contacting parts, which improves operability by extremely reducing anisotropy of torque transmitting performance due to rotational direction.

By configuring the local variation of the angle of the slit to the spiral inclination angle to be over 90°, the step parts are easily allowed to latch against the contacting parts when the tubular body is twisted to unwind the spiral of the slit, thus reducing excessive twist to occur.

By configuring the inclination angle of the step parts to the axial direction to be ±5°, the step parts are arranged substantially in parallel to the axial direction, allowing the side that bends in protruding manner when the tubular body is bent, namely the step parts formed on the side which the width of the slit widens, to move relative to the contacting parts and also making it easier to return to its original position. Accordingly, it is possible to restrain the bending rigidity of the tubular body which becomes locally high when the step parts are unable to move against the contacting parts, and thus excellent flexibility can be provided.

By forming the gap in between the slit to have a constant width, the step parts formed to protrude from one side of the paired opposing surfaces comprising the slit and the contacting parts formed on the other side of the opposing surfaces to contact the step parts are formed substantially in the same shape. Accordingly, the step parts latch against the contacting parts with more ease when the tubular body is twisted in the direction to unwind the spiral of the slit, and thus ensures to restrain excessive twist to occur.

By arranging the step parts which are formed on each slit aligned adjacent to one another in the axial direction of the tubular body by drawing a spiral in different position in the circumferential direction of the tubular body, the step parts of the adjacent slit do not overlap with one another in axial direction of the tubular body, which makes it more difficult for the bending rigidity of the tubular body to be biased depending on the circumferential position and hence provides excellent flexibility.

By comprising the tubular body with the portion having gradually changing spiral inclination angle, and by comprising the step parts that are plurally provided on said portion to the axial direction to be gradually longer from the portion where the spiral inclination angle is relatively small toward the portion where it is large, it becomes possible to achieve sufficient push-in property by the portion with larger spiral inclination angle and high bending rigidity, while high reachability and operability is ensured with a flexible portion having smaller spiral inclination angle which can easily pass through bending parts and the like in the lumen of a living body. Furthermore, since a gradual change of the spiral inclination angle of the slit leads to a gradual decline in bending rigidity, stress does not concentrate on a single spot even when the tubular body is rapidly bent, thus reducing the kink occurrence in the medical long length body.

By forming the protruding parts to protrude toward either distal end side or the base side of the tubular body with larger spiral inclination angle of the slit, a sufficient length can be assured for forming the protruding parts in the direction that the protruding parts extend in the portion where the spiral inclination angle changes.

By forming the protruding parts with narrower width toward the protruding direction, the protruding parts can part from the recessed parts and return to original position relative to the recessed parts, and it becomes possible to control the bending rigidity of the tubular body from becoming locally high and thus provide excellent flexibility.

By forming the portion of the step parts that the angle of the slit changes locally to have curvatures, the slit can be fabricated in a constantly moving manner without stopping the laser and the like against the tubular body when making the slit with a laser and the like. Accordingly, unnecessary influence on the tubular body by heat generated in the laser processing which causes the tubular material to deteriorate or deform is prevented. Furthermore, it improves safety as the portion is formed with curvatures without sharp edges.

By further comprising the tubular body with a temperature measuring part for measuring temperature on its distal end part, the temperature provided by the heat element can be monitored while the heat element can be controlled based on the temperature to be measured, and thus safe and appropriate ablation can be performed.

By further comprising the tubular body with a traction wire which passes through the inside of the tubular body with its end part connected to the distal end side of the tubular body, and can bend the tubular body by towing its base part, the tubular body can be bend freely by towing the traction wire to set the heat element in a desired position, and thus safe and appropriate ablation can be performed.

If the ablation catheter is designed to be inserted in a renal artery to ablate renal sympathetic nerve, the renal sympathetic nerve surrounding the renal artery can be efficiently ablated from within the renal artery to achieve blood pressure lowering effect.

### Brief Description of the Drawings

Fig. 1 is a plane view of the system including the ablation catheter according to the embodiment of the present invention.
Fig. 2 is a cross sectional view of an ablation catheter according to the embodiment of the present invention.
Fig. 3 is a cross sectional view of the tip side of the shaft.
Fig. 4 is a cross sectional view taken along the line 4-4 in Fig.3.
Fig. 5 is a plane view of the shaft body.
Fig. 6 is a plane view of an enlarged part of the shaft body.
Fig. 7 is a plane view of a variation of the shaft body.
Fig. 8 is a plane view of an enlarged part of the shaft body illustrating a state when the torque acts on the shaft.
Fig. 9 is a schematic cross sectional view illustrating a state when ablation is performed to the first target position by the ablation catheter.
Fig. 10 is a schematic cross sectional view illustrating a state when ablation is performed to the second target position by the ablation catheter.
Fig. 11 is a schematic cross sectional view taken along the line C-C in Fig. 10.
Fig. 12 is a plane view of another variation of the shaft body.
Fig. 13 is a plane view of an enlarged distal end of the shaft body in Fig. 12.
Fig. 14 is a plane view illustrating comparative examples of the shaft body.

### Description of Embodiments

A preferable embodiment will be recited and described in detail below referring to attached drawings. Note that dimension ratios of the drawings may be exaggerated for convenience of explanation, and may differ from the actual ratio.

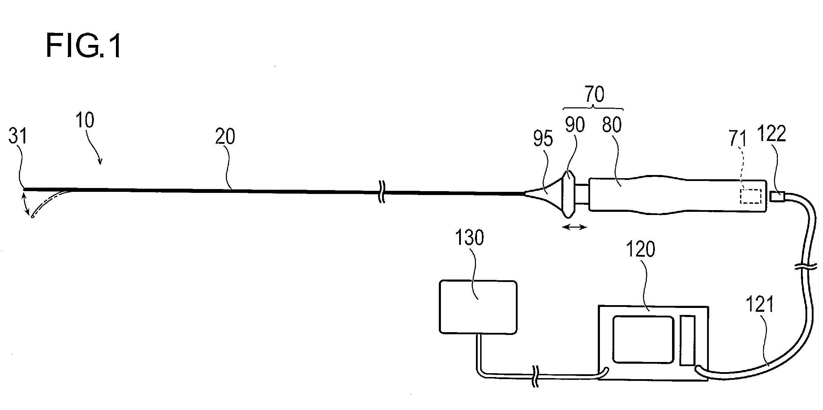

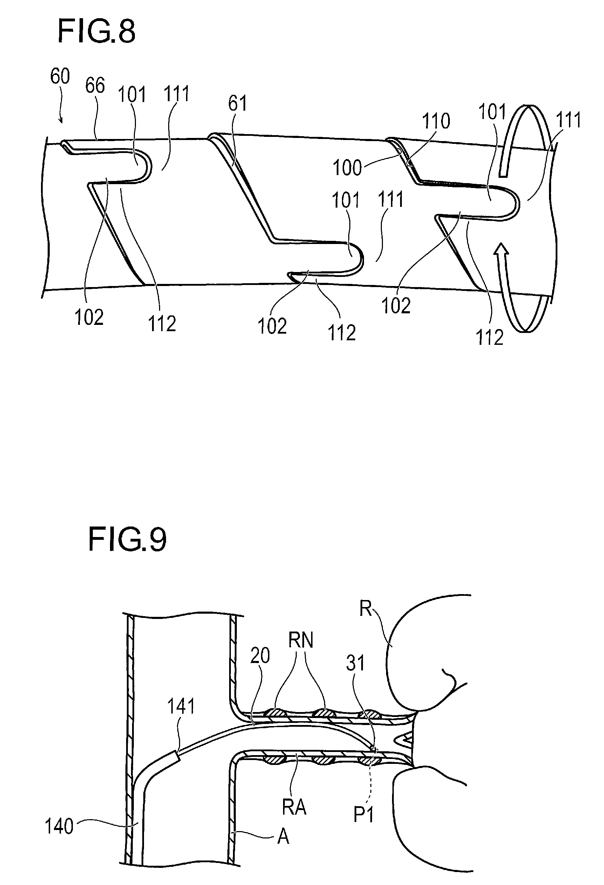

An ablation catheter 10 according to the present embodiment is introduced to a renal artery RA transdermally to ablate (cauterize) renal sympathetic nerve RN (refer to Fig. 9) runs surrounding the renal artery RN, and as illustrated in Fig. 1 to 3, it is comprised of a long shaft 20, a heating part 30 (heat element) to carry out heating for ablation, a temperature measuring part 40 for measuring temperature, a traction wire 50 for bending the shaft 20, an operation part 70 provided on the base part of the shaft 20 to carry out operation and an anti-kink tube 95 provided on the connecting part of the shaft 20 and the operation part 70. Note that, in the specification below, the side inserted in a lumen will be referred to as distal end or distal end side and the hand side to be operated will be referred to as base or base side.

The shaft 20 is comprised of a tubular shaft body 60 (tubular body) extending to the axial direction X and a coating part 67 covering the shaft body 60.

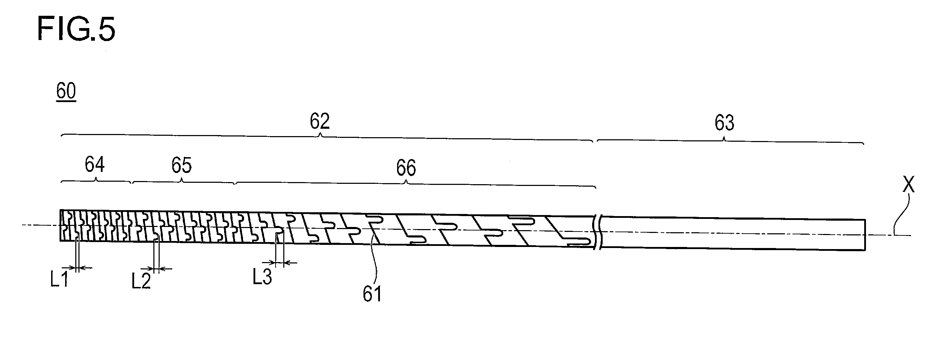

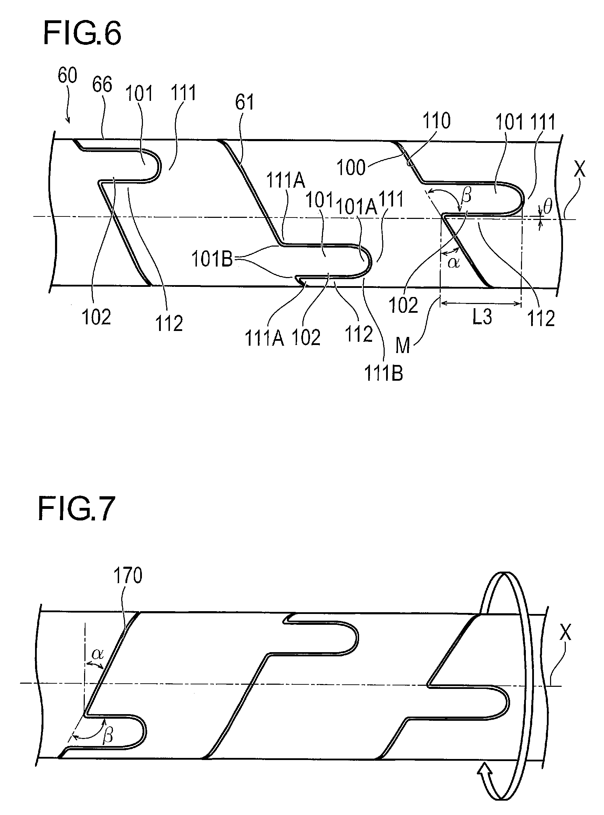

As can be seen in Fig. 5 and 6, the shaft body 60 includes a flexible part 62 on a distal end side with a spiral slit 61 formed thereon and a high rigidity part 63 at a base side formed without slit 61. The flexible part 62 consists of the first flexible part 64 positioned closest to the distal end side, the second flexible part 65 provided at the base side of the first flexible part 64 and the third flexible part 66 provided at the base side of the second flexible part 65. The slit 61 is fabricated by spiral slit processing using techniques generally performed in laser processing and the like. Further, as illustrated in Fig. 7, spiral cut processing can be applied to fabricate a slit 170 in the direction opposite to the slit 61 shown in Fig. 5 and 6.

On the first flexible part 64, the slit 61 is formed in a predetermined pitch, while on the second flexible part 65 the slit 61 is formed in a predetermined pitch wider than in the first flexible part 64. On the third flexible part 66, the slit 61 is formed so that a pitch becomes gradually wider from the side of the second flexible part 65 toward the base side. Note that the slit 61 formed on the first flexible part 64, second flexible part 65 and third flexible part 66 is formed as a single continuous slit 61. Here, the pitch refers to an interval between the 2 adjacent slits formed on the tubular body.

A spiral inclination angle α, which is an angle of extending direction of the spiral of the slit 61 to a cross section orthogonal to the axial direction X, varies respectively in the first flexible part 64, second flexible part 65 and third flexible part 66. Normally, the spiral inclination angle α becomes smaller as the pitch of the slit 61 is narrower, and larger as the pitch of the slit 61 is wider.

The flexible part 62 of the shaft body 60 is reduced of the bending rigidity to have an easily bend, flexible structure by having the slit 61 formed thereon. Since the first flexible part 64 is formed with the slit 61 with the narrowest pitch, the bending rigidity thereof is lower than that of the second flexible part 65 and the third flexible part 66. Since the second flexible part 65 is formed with the slit 61 with a pitch wider than the first flexible part 64 but narrower than the third flexible part 66, the bending rigidity thereof is higher than that of the first flexible part 64 but lower than that of the third flexible part 66. Since the third flexible part 66 is formed with the slit 61 having a pitch that gradually becomes narrower toward the distal end side, the bending rigidity thereof becomes lower toward the distal end side. With such structure of the shaft body 60 provided with sufficient rigidity on the base side and with more flexibility toward the distal end side, the ablation catheter 10 is allowed a sufficient push-in property by the base side portion having high rigidity, while high reachability and operability can be obtained by the flexible distal end side portion having low rigidity which allows to move easily even through the bending parts of the biological lumen. Further, since the pitch of the slit 61 changes gradually and the rigidity of the third flexible part 66 decreased gradually toward the distal end side, stress may not concentrate on a single part even when the shaft body 60 is rapidly bend and can reduce kink occurrence on the shaft 20.

The slit 61 is comprised of a pairing opposing surfaces 100, 110 arranged opposed to each other, with a plurality of protruding parts 101 protrudingly formed on one of the opposing surface 100, and a plurality of recessed parts 111 that the protruding parts enter in provided on the other opposing surface 110. The protruding parts 101 are provided with step parts 102 formed in stepped shape with the slit 61 changing its angle locally against the spiral inclination angle α. As illustrated in Fig. 7, the step parts 102 contact the contacting parts 112 that are arranged opposite the step parts 102 when the shaft body 6 is twisted to the direction to unwind the spiral of the slit 61 (refer to a void arrow when the tubular body is operated from the side of an arrow shown in Fig. 6), and thus able to reduce excessive twist. In other words, the step parts 102 are the parts where the angle of the slit 61 against the spiral inclination angle α provided on the protruding parts 101 changes locally to form stepped shape, as well as the parts contacting the recessed parts 111 when the tubular body having the slit 61 is twisted to the direction to unwind the spiral. As illustrated in Fig. 7, the slit 170 can be formed in the direction opposite of Fig. 6. In such case, the direction to unwind the spiral of the slit 170 would be opposite to the direction in Fig. 6.

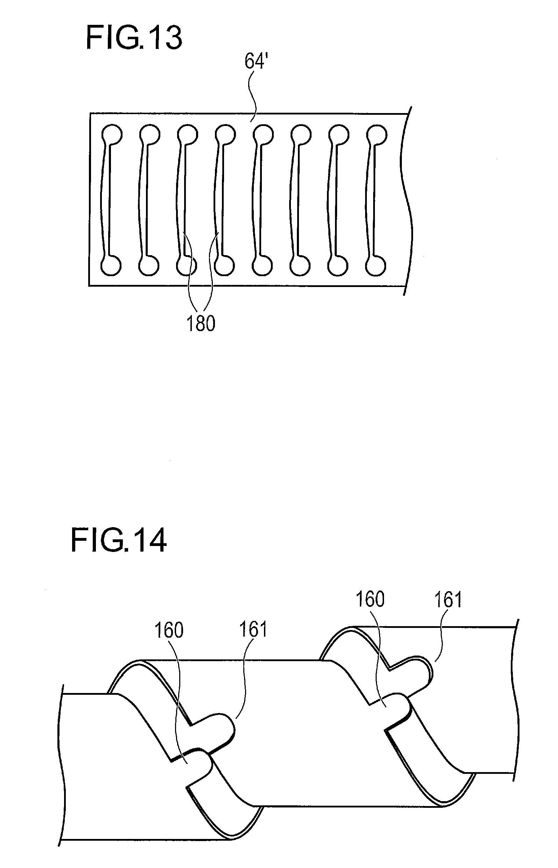

Lengths L1∼L3 of the step parts 102 to the axial direction X are, as illustrated in Fig. 5 and 6, formed longer in the portions where the pitch of the slit 61 is relatively large than in the portions where the pitch is small. Accordingly, the length L2 of the step parts 102 provided on the second flexible part 65 having the pitch of the slit 61 wider than the first flexible part 64 is longer than the length L1 of the step parts 102 provided on the first flexible part 64. Further, the length L3 of the step parts 102 provided on the third flexible part 66 having the pitch of the slit 61 wider than the second flexible part 65 is longer than the length L2 of the step parts 102 provided to the second flexible part 65. Further, the length L3 of the step parts 102 provided on the third flexible part 66 where the pitch is wider toward the direction of the base side is longer in the step parts 102 that are provided near the base side. That is to say that the step parts 102 provided to the portion with wider pitch and larger spiral inclination angle α are formed with longer length L1∼L3. As illustrated in the comparative examples in Fig. 14, the protruding parts 160 on the portion having wider pitch and larger spiral inclination angle α tend to separate completely from the recessed parts 161 making it difficult to return back to the original position, whereas in the present embodiment, the lengths L1∼L3 of the step parts 102 are formed longer in the step parts 102 that are provided to the portions with larger spiral inclination angle α, which makes it more difficult for the protruding parts 101 to separate from the recessed parts 111 in proportion to the level of spiral inclination angle α. Here, the length of the step parts 102 in the axial direction X indicates the distance between a line orthogonal to the axial direction X which contact the protruding parts 101, and a line orthogonal to the axial direction X that passes through the portion where the angle of the slit 61 changes locally on the opposing surfaces 100 of the slit 61 with the spiral inclination angle α.

Further, the width of the protruding parts 101 is formed to be gradually narrower toward the protruding direction in the distal end part at the protruding direction. This allows the protruding parts 101 to move to the direction to exit from the recessed part 111 as well as to return to the original position against the recessed part 111, reducing bending rigidity of the shaft body 60 to be locally high and providing excellent flexibility.

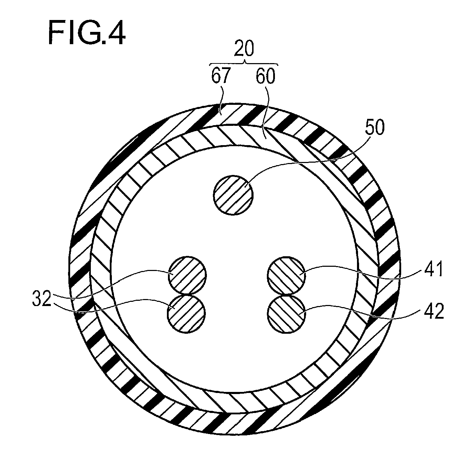

Further, the local variation 6 of the angle of the slit 61 of the step parts 102 may preferably exceed 90°. Specifically, it is preferable to form the angle of the step parts 102 of the protruding parts 61 against the opposing surfaces 100 of the slit 61 with the spiral inclination angle α to exceed 90°. With such structure, the step parts 102 provided to the protruding parts 101 can be easily caught on the contacting parts 112 provided to the recessed parts 111 as illustrated in Fig. 4 when the base shaft 60 is twisted to the direction to unwind the spiral of the slit 61, and more reliably restrains excessive twist. Moreover, it allows to restrain the protruding parts 101 from exiting from the recessed parts 102 when the base shaft 60 is twisted to the direction to unwind the spiral. Here, to achieve the above mentioned effect for sure, it is preferable that the angle formed with the contacting parts 112 of the protruding parts 61 and the opposing surfaces 110 of the slit 61 with the spiral inclination angle α does not exceed 90°. The local variation 6 of the angle of the slit 61 does not need to be over 90°.

Furthermore, the step parts 102 may preferably be formed to be substantially parallel to the axial direction X, with the inclination angle θ to the axial direction X to be at least within ±5°. By forming the step parts 102 to be substantially parallel to the axial direction X in at least within ±5°, the side that bends in convex when the shaft body 60 is bent, namely the protruding parts 101 provided to the side of which the width of the gap between the slit 61 widens, can easily move within the recessed parts 111 to separate therefrom as well as easily return back to the original position relative to the recessed parts 111. Thereby, the bending rigidity of the shaft body 60 can be restrained from getting locally high when the protruding parts 101 can't part from the recessed parts 111, and provides excellent flexibility. Further, it is more preferable for the step parts 102 to be formed parallel to the axial direction X as the protruding parts 101 provided to the side which the width of the gap between the slit 61 widens, can move within the recessed parts 111 to easily part therefrom. The inclination angle θ of the step parts 102 to the axial direction X step parts 102 does not have to be within ±5°.

Furthermore, the slit 61 may preferably be formed so that the width of the gap between the slit 61 is constant. According to this structure, outer contour shape of the protruding parts 101 and the recessed parts 111 become substantially same. Thereby, as illustrated in Fig. 4, the step parts 102 provided to the protruding parts 101 can be easily caught on the contacting parts 112 provided to the recessed parts 111 when the base shaft 60 is twisted to the direction to unwind the spiral of the slit 61, and thus restraining the excessive twist for sure.

By drawing the spiral, the step parts 102 provided to the respective slit 61 lined adjacent in the axial direction X are arranged in different position relative to circumferential direction of the shaft body 60, so that the step parts 102 adjacent to each other do not overlap in the axial direction X. To be more specific, at least 2 continuous adjacent step parts 102 do not overlap with each other in the axial direction X. For example, the step parts 102 can be formed in every 125°, 270° or 450° in circumferential direction of the shaft body 60 along the spiral. Accordingly, the bending rigidity of the shaft body 60 does not be biased depending on the position in circumferential direction and thus provides excellent flexibility. Further, the step parts 102 do not have to be formed at every same angle to the circumferential direction of the shaft body 60. Further, all of the step parts 102 may be formed not to overlap in the axial direction of the shaft body 60.

The protruding parts 101 are formed to protrude toward either the distal end side or the base part side with a larger spiral inclination angle α of the slit 61, namely toward the base part side which the pitch widens. Thereby, sufficient length can be ensured to form the protruding parts 101 in the extending direction of the protruding parts 101 on the portion where the pitch of the slit 61 changes. Further, the protruding parts may be formed to protrude toward the direction of either the distal end side or the base part side that has a narrower pitch of the slit. Furthermore, the direction of protruding parts may vary in respective protruding parts.

As illustrated in Fig. 6, portions where the angle of the slit 61 changes locally, namely, edge parts 101A that form protrusion at the edge of the protruding part 101 and corner parts 101B that form recess, as well as edge parts 111A that form protrusion at the edge of the recessed parts 111 and corner parts 111B that form recess are formed to have curvatures. This way, laser and the like does not need to be stopped against the tubular body to be processed when machining the above mentioned edge parts 101A, 111A and the corner parts 101B, 111B when forming the slit 61 by the laser and the like. Thereby, laser and the like is constantly moved against the tubular body while processing, which prevents unnecessary heat generated by laser processing to be added to the edge parts 101A, 111A and the corner parts 101B, 111B and restrains the material of the tubular body to deteriorate or deform. Further, as the edge parts 101A, 111A and the corner parts 101B, 111B are formed with curvatures, sharp edges do not exist which improves safety.

It is preferable that material with relatively high rigidity, such as metals like Ni-Ti, brass, SUS and aluminum may be used for shaft body 60. As long as the material is relatively high in rigidity, resins such as polyimide, vinyl chloride, polycarbonate may also be used.

Size of the high rigidity part 63 of the shaft body 60 is not limited but its outer diameter is approximately 0.5mm∼3.0mm and its thickness is approximately 100µm∼300µm.

Size of the first flexible part 64 of the shaft body 60 is not limited but its outer diameter is approximately 0.5mm∼3.0mm, thickness is approximately 100µm∼300µm and pitch should be approximately 0.2mm∼1.0mm.

Size of the second flexible part 65 of the shaft body 60 is not limited but outer diameter is approximately 0.5mm∼3.0mm, thickness is approximately 100µm∼300µm and pitch should be approximately 1.0mm∼2.0mm.

Size of the third flexible part 66 of the shaft body 60 is not limited, but outer diameter is approximately 0.5mm∼3.0mm, thickness is approximately 100µm∼300µm and pitch should be approximately 2.0mm∼5.0mm.

Although it is not limited, the width of the gap of the slit 61 is approximately 0.01mm∼0.05mm.

Although it is not limited, coating part 67 is preferably made of material with insulation property, and polymer material such as polyolefin (for example, polyethylene, polypropylene, polybutene, ethylene-propylene copolymer, ethylene-vinylacetate copolymer, ionomer or mixture of more than 2 of the above), crosslinked polyolefin, polyvinyl chloride, polyamide, polyamide elastomer, polyester, polyester elastomer, polyurethane, polyurethane elastomer, fluororesin, polyimide or mixture thereof may be used.

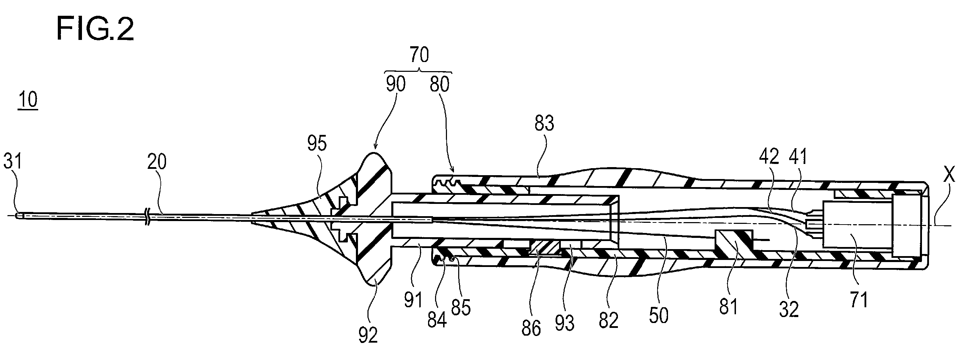

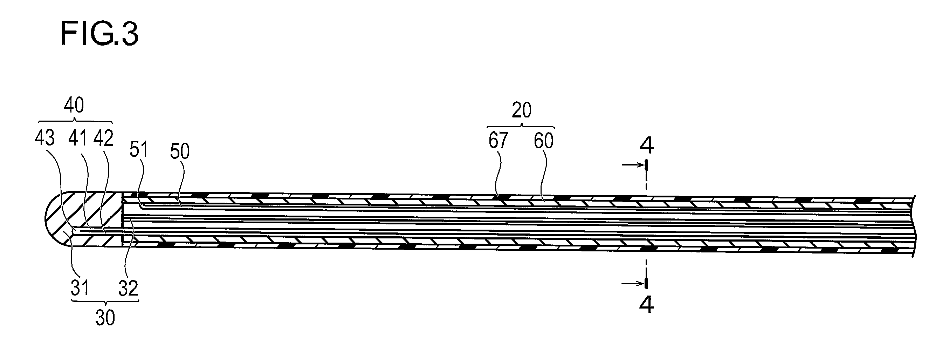

As illustrated in Fig. 2 and 3, the heating part 30 is provided with a single polar (monopolar) electrode tip 31 (heat element) and a lead wire 32 for supplying current to the electrode tip 31. The base side of the electrode tip 31 is joined to the distal end part of the shaft 20 and its distal end side is formed in a shape of hemisphere. Damage to the biological tissue that comes in contact can be reduced by having the distal end side of the electrode tip 31 made in a hemispherical shape. The lead wire 32 penetrates through the shaft 20 with its distal end side joined to the electrode tip 31 and its base side joined to one of the terminals of a connector 71 formed on the operation part 70. The heating part 30 can induce thermal neural modulation to fibers of the renal sympathetic nerve RN to generate, for example, necrosis, thermal deterioration or peeling to the nerve fibers. 35° to 85° in Celsius would be preferable heating temperature by the heating part 30, but it is not limited thereto.

A temperature measuring part 40 is provided to monitor and control the heating by the heating part 30. The temperature measuring part 40 is, for example, a thermocouple having 2 different types of metal wires 41, 42 with its contact at the distal end fixed to the distal tip 31. As long as it is capable of measuring temperature, the temperature measuring part 40 does not have to be thermocouple, and may be, for example, thermostat and the like. The respective base parts of the metal wires 41, 42 of the thermocouple are joined with different terminals of the connector 71 provided to the operation part 70.

Although it is not illustrated, other than the temperature measuring part 40, an impedance sensor for checking contact state of the electrode tip 31 and biological tissue may be provided to the ablation catheter.

The distal end of wire 51 at the distal end side of traction wire 50 is fixed to one side of the inner wall surfaces of the distal end part of the shaft 20 and base side thereof is fixed to the wire retaining part 81 provided to the operation part 70. Accordingly, by towing the side of the shaft 20 with the distal end of the wire fixed thereto, the traction wire 50 is pulled by the operation part 70 to allow a portion including the flexible part 62 at the distal end part of the shaft 20 extending substantially straight to bend, and by loosening the traction by the traction wire 50, the distal end part of the shaft 20 is allowed to return to its substantially linear original shape due to elasticity of the flexible part 62 itself.

As illustrated in Fig. 1 and 2, the operation part 70 is a portion with which the operator operates the ablation catheter, and it is provided with a operation body 80 retained by the operator and a sliding part 90 which may slide against the operation body 80 in the axial direction.

The operation body 80 is substantially tubular with an inner tubular body 82 which comes in contact with the sliding part 90 slidably and accommodates connector 71 and the like, and an outer tubular body 83 covering outer periphery of the inner tubular body 82. Screw grooves 85 are formed on the inner peripheral surface of the distal end side of the outer tubular body 83, and the outer tubular body 83 and the inner tubular body 82 can be fixed to each other by screwing screw threads 84 formed on the outer peripheral surface of the distal end side of the inner tubular body 82 to the screw grooves 85.

Inside the inner tubular body 82A, a wire retaining part 81 which is fixed to the base side of the traction wire 50 is formed. To the base side of the inside of the inner tubular body 82, a connector 71 is fixed. The connector 71 is provided with terminals that the metal wires 41, 44 of the thermocouple and the lead wire 32 are electrically connected respectively. The connector 71 may be connected to a supply side connector 122 of a cable 121 extending from an energy supplying device 120 which is installed externally. Thusly, current can be supplied to the lead wire 32 connected to the connector 71 via the cable 121 and the supplying side connector 122, while voltage generated in the thermocouple connected to the connector 71 may be measured.

To the inner tubular body 82, a guide pin 86 is fixed to protrude from the inner periphery surface which slides against the sliding part 90.

The sliding part 90 is provided with a sliding inner tubular part 91 accommodated inside the distal end side of the inner tubular body 82 in a slidable manner, and a substantially disk-shaped sliding operation part 92 formed on the distal end side of the sliding inner tubular part 91 to be operated by the operator. The sliding operation part 92 is formed with a larger diameter than the operation body 80 to make it easier for the operator to hold and operate the operation body 80.

On the outer peripheral surface of the sliding part 90 that slides against the inner tubular body 82, a guiding groove 93 is formed to extend to the axial direction X, and in the guide groove 93, the guide pin 86 projecting from the inner tubular body 82 is accommodated slidably to the axial direction X. Thusly, the sliding part 90 is allowed to move to the axial direction X against the operation body 80, while being controlled to move against the operation body 80 to the rotational direction.

Preferably, as a material used for the inner tubular body 82, the outer tubular body 83 and the sliding part 90, resins such as ABS resin, polycarbonate, polyamide, polysulfone, polyarylate and methacrylate-butylene-styrene copolymer may be used. Further, any of the above materials may be optionally combined for use.

The anti-kink tube 95 is mounted to the distal end side of the sliding operation part 92 and outside of the shaft 20 to prevent kink (bending) of the shaft 20 close to the distal end part of the sliding part 90.

The energy supplying device 120 is allowed to supply electrical energy of high frequency to the heating part 30 for ablating biological tissue via the cable 121 and the supply side connector 122, and also allowed to measure voltage produced at the thermo couple of the temperature measuring part 40. A calculating means such as computer is connected or built within the energy supplying device 120, and thus able to control the heating temperature, heating time and such while monitoring the heating temperature by the heating part 30. Electrical energy to be supplied is 0.1W to 8.0W preferably, but it is not limited thereto.

Further, a return electrode 130 that is magnetically opposite to the electrode tip 31 and attached to a body surface to disperse and recover current is connected to the energy supplying device. Ablation does not necessary have to be performed by electrical energy, and microwave energy, ultrasonic energy, coherent light such as laser, incoherent light, heated fluid, cooled fluid and such may be performed. Ablation may be performed not only by heating but also by cooling.

The usage of the ablation catheter according to the present embodiment is explained in detail below.

Firstly, the supply side connector 122 of the cable 121 extending from the energy supplying device 120 is connected to the connector 71 and the return electrode 130 is attached to the body surface of a patient. Then the traction wire 50 is loosened by moving the sliding part 90 closer to the operation body 80 so that the distal end part of the ablation catheter 10 becomes substantially straight.

Next, a femoral artery of the thigh is exposed and a sheath (not illustrated) is fixed transdermally by well known art. Then the guide wire (not illustrated) is inserted into the femoral artery via the sheath and introduced to the vicinity of a renal artery RA of a main artery A through the femoral artery to a iliac artery. A guiding catheter 140 is inserted along the guide wire to guide a tip opening 141 of the guiding catheter 140 to the entrance of the renal artery RA as illustrated in Fig. 9. Then, the guide wire is removed. The guide wire can be introduced from a vein other than the femoral artery such as radial artery, brachial artery and axillary artery.

The shaft 20 of the ablation catheter 10 is inserted into the guiding catheter 140 from the base side of the guiding catheter 140, and the shaft 20 is projected out from the tip opening 141 of the guiding catheter 140 to be insert inside the renal artery RA leading to a kidney R. By having the high rigidity part 63 formed without the slit provided to the shaft body 60, high push-in and torque transmitting property may be obtained while obtaining high reachability and operability by easily passing through even the bending part of the vein with the flexible part 62. Particularly, the entrance to the renal artery RA from the main artery A is bent largely, but since the third flexible part 66 of the shaft body 60 is mainly positioned in this portion, the shaft 20 is inserted to transmit torque to the distal end side as it bends flexibly.

Next, while adjusting the axial directional position of the electrode tip 31 by operating the operation body 80 and the sliding part 90, the retraction wire 50 is pulled by either moving the sliding part 90 against the operation body 80 toward the distal end side or moving the operation body 80 against the sliding part 90 towards the base side. Accordingly, mainly the portions at the distal end side of the shaft 20 where the first flexible part 64 and the second flexible part 65 are formed are bent flexibly, so that the electrode tip 31 comes in contact with the first target position P1 adjacent to the renal sympathetic nerve RN.

Then, electrical energy of high frequency is supplied to the electrode tip 31 from the energy supplying device 120, and since the return electrode 130 is attached to the body surface, biological tissue nearby the electrode tip 31 is heated. Thereby, fibers of the renal sympathetic nerve RN positioned close to the electrode tip 31 undergo necrosis, thermal alteration or peeling, for example.

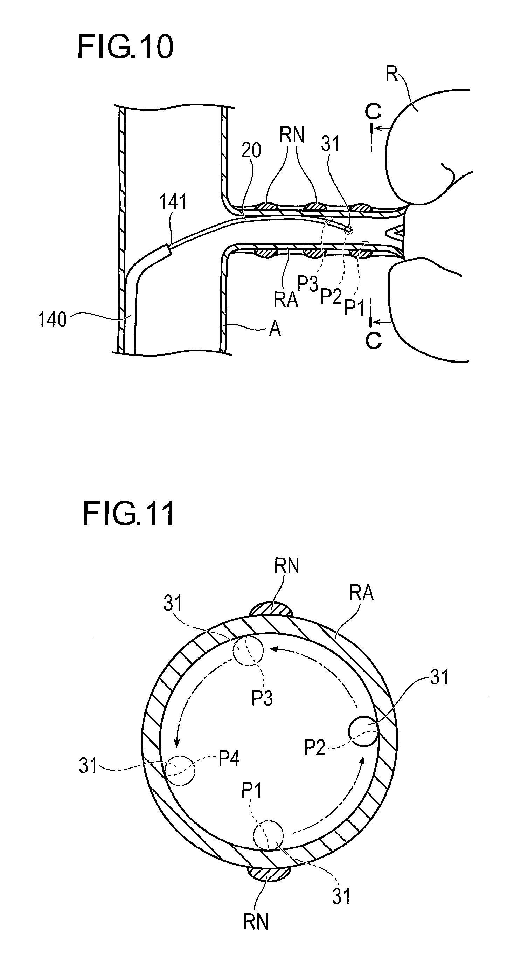

Next, as illustrated in Fig. 10 and 11, the operation part 70 is operated to move the electrode tip 31 along the longitudinal axis of the renal artery RA to a different position and also move it to a different position to the circumferential direction to bring the electrode tip 31 in contact with the second target position P2 which is adjacent to the renal sympathetic nerve RN. When moving the electrode tip 31, the sliding part 90 is drawn close to the operation body 80 to loosen the traction wire 50, so that the distal end of the shaft 20 becomes substantially straight, and the operation part 70 as a whole is moved to the axial direction X to move the electrode tip 31 along the longitudinal axis of the renal artery RA. Further, by rotating the operation part 70 along the central axis, the electrode 31 is rotated due to the torque transmitted by the shaft 20. Then the traction wire 50 is pulled by either moving the sliding part 90 toward the distal end side against the operation body 80 or moving the operation body 80 toward the base side against the sliding part 90. Thereby, mainly the portion of the shaft 20 at the distal end side where the first flexible part 64 and the second flexible part 65 are provided bends flexibly to bring the electrode tip 31 in contact with the second target position P2 adjacent to the renal sympathetic nerve RN. Then, the electrical energy of high frequency is supplied to the electrode tip 31 from the energy supplying device 120 again to cause necrosis, thermal alteration or peeling of the fibers of the renal sympathetic nerve RN located in the vicinity of the electrode tip 31. Thusly, by moving it along the longitudinal axis of the renal artery RA while also moving it to a different position in circumferential direction, it becomes possible to cause necrosis, thermal alteration or peeling to the renal sympathetic nerve RN localized in and outside the outer membrane of the renal artery RA spatially in the entire periphery within the renal artery RN. Since the renal artery RN may not be damaged in its entire periphery in the section within the renal sympathetic nerve RN, risk of vasoconstriction may be reduced.

Further, while moving the electrode tip 31 sequentially to positions P3, P4 ... that are different along the longitudinal direction of the renal artery RA and also different in the circumferential direction, the electrical energy of high frequency is supplied again to the electrode tip 31 to cause necrosis, thermal alteration or peeling to the fibers of the renal sympathetic nerve RN located in the vicinity of the electrode tip 31. In the present embodiment, the electrode tip 31 is moved by approximately 0.3mm to 1.0mm along the longitudinal direction of the renal artery RA while rotated by 90° to 270° in the circumferential direction, and creates approximately 4 to 6 damaged portions in spots so as to draw a spiral on the inner wall surface of the renal artery RA. The heated position and number of spots to be heated does not have to be in the range mentioned above.

The above treatment is carried out to both left and right side of the renal artery RA and then the ablation catheter, guiding catheter 140 and sheath are removed to complete the procedure.

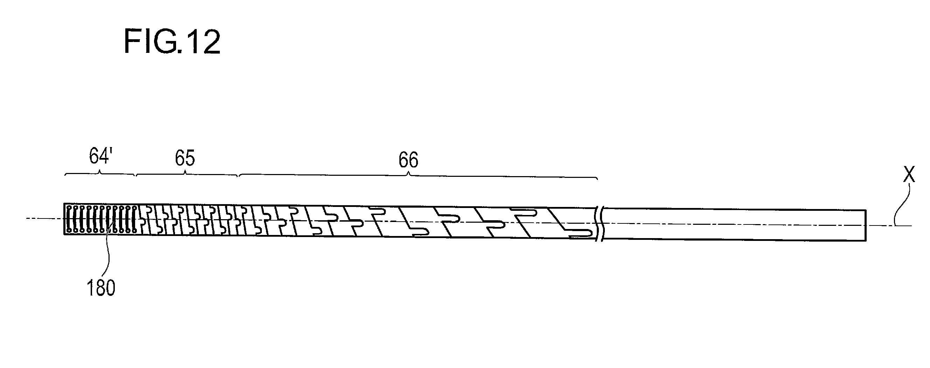

In the above embodiment, the shaft body 60 is comprised of the high rigidity part 63, the first flexible part 64, the second flexible part 65 and the third flexible part 66, as illustrated in Fig. 5, with the spiral slit according to the present invention formed on the first flexible part 64, the second flexible part 65 and the third flexible part 66, but the invention is not limited thereto. For example, in the shaft body 60 as illustrated in Fig. 5, since the first flexible part 64 is flexible towards all directions, it is difficult to predict to which direction the first flexible part 64 will bend by pulling the traction wire 50, and therefore it is difficult to predict with which position within the renal artery RA the electrode tip 31 will come in contact. Hence, as illustrated in Fig. 12, it is preferable to configure it so that the first flexible part 64' only bends to a certain direction by applying processing to only one side of the tubular body. With such configuration, it becomes possible to bend the electrode tip 31 to the desired direction to be bent when the operation body 80 is moved against the sliding part 90 toward the base side to pull the traction wire 50. Thereby, it is easier to bring the electrode tip 31 in contact with the target portion within the renal sympathetic nerve RN.

To be more specific, apart from the configuration of the first flexible part 64', the configuration in Fig. 12 is comprised of the high rigidity part 63, the second flexibility part 65 and the third flexibility part 66 in Fig. 5. For example, the first flexible part 64' in Fig. 12 is formed with slit 180 to only one side of a tubular body as illustrated in Fig. 13. The width of the gap of the slit 180 is not particularly limited but is approximately 0.05mm to 0.3mm. Although the size is not particularly limited, the flexible part of a shaft body has outer diameter of approximately 0.5mm to 3.0mm, the thickness of approximately 50µm to 300µm and the gap between the slit 180 is approximately 0.2mm to 1.0mm. With such configuration, the side formed with the slit 180 bends inward to contracts when the traction wire is retracted. In case of forming the slit 180 on the first flexible part 64', it is possible to adjust the angle the first flexible part 64' bends toward the axial center of the shaft body by adjusting shape and width of the slit 180 on the first flexible part 64'. It is preferable to set the angle of the first flexible part 64' to the axial center of the shaft body to be below the range of 45° to 90° to allow easier contact with the inner wall surface of the renal artery, If the first flexible part 64' bends more than 90°, the base part is more likely to contact the inner wall surface of the renal artery than the electrode tip (not illustrated) at the distal end of the first flexible part 64', hence making it difficult for the electrode tip to contact the renal artery, and ablation of the renal sympathetic nerve may be more difficulty to performing.

As above, operability of the ablation catheter 10 according to the present invention can be improved by comprising the shaft body 60 (tubular body) with portions having different spiral inclination angle α of the slit 61 to optionally set the flexibility depending on the position of the axial direction X. Further, since the step parts 102 where the angle to the spiral inclination angle α changes locally are provided to one of the pairing opposing surfaces 100, 110 comprising the slit 61 of the shaft body 60 and the contacting parts 112 where the step parts 102 contact when the shaft body 60 is twisted to the direction to unwind the spiral of the slit 61 are provided to the other opposing surface 110, the step parts 102 contact the contacting parts 112 when it is twisted to the direction to unwind the spiral, preventing the gap between the slit 61 to open. Furthermore, since the lengths L1~L3 of the step parts 102 toward the axial direction X are formed longer in the portions where the spiral inclination angle α of the slit 61 is relatively large than in the portions where it is small, the portions where the latching of the step parts 102 to the contacting parts 112 tends to disengage easily due to having larger spiral inclination angle α of the slit 61 are made with longer length to be more difficult to disengage from the contacting parts 112, which allows to improve operability by extremely reducing anisotropy of torque transmitting performance by rotational direction.

Further, it is also possible to prevent the slit from opening, for example, by forming the opposing surfaces that comprise the slit partly connected so that the slit is in cut line form, but in such case, stress tends to concentrate on the portions connecting the opposing surfaces and may lead to deformation, fracture or unexpected operation due to abrasion of material. On the other hand, in the present embodiment, deformation, fracture or unexpected operation due to abrasion of material is prevented as the slit 61 is formed continuously without cut line.

Moreover, since the local variation 6 of the angle of the slit 61 to the spiral inclination angle α exceeds 90°, the step parts 102 are allowed to latch more easily to the contacting parts 112 when the shaft body 60 is twisted to the direction to unwind the spiral, and restrains excessive twist for sure.

Furthermore, since the inclination angle θ of the step parts 102 to the axial direction X is within ±5°, the step parts become substantially in parallel to the axial direction X, allowing the step parts formed on the side which bends in convex shape when the shaft body 60 is bend, namely the side which the gap between the slit 61 needs to widen, to move within the recessed parts 111 and to return easily to the recessed parts 111. Thereby, it is possible to prevent the bending rigidity of the shaft body 60 to be locally high due to the protruding parts not being able to move apart from the recessed parts 111, and hence provide excellent flexibility.

Since the width of the gap between the slit 61 is formed to be constant, shape of the step parts 102 formed to protrude from one side of the pair of opposing surfaces 100, 110 and shape of the contacting parts 112 formed on the other side of the opposing surfaces 100, 110 to contact the step parts 102 are formed substantially the same. Therefore, the outer shape of the protruding parts 101 formed to protrude from one side of the pair of opposing surfaces 100, 110 and the outer shape of the recessed parts formed to be recessed from the other side of the opposing surfaces 100, 110 become substantially the same. Accordingly, when the shaft body 60 (tubular body) is twisted to the direction to unwind the spiral, the step parts 102 formed to the protruding parts 101 engage more easily to the contacting parts 112 formed to the recessed parts 111 and prevents excessive twist for sure.

Further, since the positions of the step parts 102 provided on each of the slits 61, which are aligned adjacent to each other in the axial direction X of the shaft body 60 (tubular body) by drawing a spiral, are different in circumferential direction of the shaft body 60, the step parts 102 do not overlap with each other in axial direction X of the shaft body 60. Therefore, the bending rigidity of the shaft body 60 may not become biased depending of the position in circumferential direction, and thus provides excellent flexibility to the shaft 20.

As the shaft body 60 (tubular body) is formed with the portion where its spiral inclination angle α gradually changes, and the length L3 to the axial direction X of the step parts 102 which are plurally provided to the portion is formed to be gradually longer in the portion where the spiral inclination angle α is large than in the part where it is relatively small, sufficient push-in property may be secured with the high rigidity in the portion with larger spiral inclination angle α of the shaft body 60 while high reachability and operability may be obtained with the high flexibility in the portion with smaller spiral inclination angle α, being able to easily pass through curved parts and the like in the biological lumen. Further, since the bending rigidity gradually decreases due to the gradual change in spiral inclination angle α, the stress may not concentrate on a single point even when the shaft body 60 is bent rapidly, and reduces the occurrence of kink in the ablation catheter 10.

It becomes possible to secure sufficient length to form the protruding parts 101 in the direction that the protruding parts extend in the portion where the spiral inclination angle α changes, since the protruding parts 101 are formed to protrude toward either the distal end side or the base side which is in the direction where the spiral inclination angle α is larger (in the present embodiment, the base side).

Further, since the width of the protruding parts 101 decreases toward the protruding direction, it allows the protruding parts 101 to part from the recessed parts 111 and return to the original position in the recessed parts 111, and to restrain the bending rigidity of the shaft body 60 to be locally high, and provides excellent flexibility.

Since the portion where the angle of the slit 61 in the step parts 102 changes locally is formed to have curvatures, it becomes possible to form a slit in a constantly moving manner without stopping the laser and the like against the tubular body when forming the slit with the laser and the like. Thereby, unnecessary influence by heat generated in the laser processing to the tubular body which causes the tubular material to deteriorate or deform may be restrained. Further, since it is formed to have curvatures, sharp edges may be eliminated and improves safety.

Further, as the temperature measuring part 40 is provided to the distal end side of the shaft body 60 (tubular body), it allows to monitor the heating temperature while controlling the heating part 30 (heat element) based on the temperature measured, thereby safe and proper ablation may be conducted.

Furthermore, as the traction wire 50 which is inserted through the shaft body 60 (tubular body) with its distal end part connected to the distal end part of the shaft body 60 to be able to bend the shaft body 60 by pulling the base part is provided, the heating part 40 (heat element) is allowed to be positioned in desired position by pulling the traction wire 50 to bend the tubular body freely, thereby allowing safe and proper ablation to be conducted.

Since the ablation catheter 10 according to the present embodiment is configured to be inserted within the renal artery RA to ablate the renal sympathetic nerve RN, the renal sympathetic nerve RN positioned surrounding the renal artery RA may be ablated efficiently from the renal artery RA, to obtain excellent hypotensive effect on blood pressure.

The present invention is not limited only to the embodiment explained above and various modifications are possible within the scope of technical ideas of the present invention by a person skilled in the art. For instance, the electrode tip 31 is a monopolar electrode in the present embodiment, but it may also be configured so that the ablation catheter is a bipolar electrode.

Further, the ablation catheter 10 according to the present embodiment is provided with the shaft body 60 (tubular body) having 3 parts, namely the first flexible part 64, the second flexible part 65 and the third flexible part 66 with different spiral inclination angle α of the slit 61, but the configuration is not limited thereto as long as it is formed with a portion where the spiral inclination angle α differs. Accordingly, for example, the portion like the third flexible part 66 where the spiral inclination angle α of the slit 61 gradually changes does not need to be provided, or it can be made only from the portion like the third flexible part 66 where the spiral inclination angle α of the slit 61 gradually changes. Further, the spiral inclination angle α of the slit 61 becomes smaller toward the distal end side in the ablation catheter 10 of the present embodiment, but positional relation of the portions having different spiral inclination angle α of the slit 61 is not particularly limited and, for example, the spiral inclination angle α may be larger toward the distal end side, or the portion having large spiral inclination angle α and portion having smaller spiral inclination angle may be alternately arranged. Furthermore, direction of the spiral of the slit is not limited, or it may have multiple spiral structure with plurality of slits.

Further, although the ablation catheter 10 according to the present embodiment is for ablating renal sympathetic nerve RN, it may also be used for ablating biological tissue other than renal sympathetic nerve RN.

### Descriptions of symbols

- 10: ablation catheter
- 20: shaft
- 30: heating part (heat element)
- 40: temperature measuring part
- 50: traction wire
- 60: shaft body (tubular body)
- 61,171: slit
- 62: flexible part
- 63: high rigidity part
- 64: first flexible part
- 65: second flexible part
- 66: third flexible part
- 90: sliding part
- 100,110: opposing surface
- 101: protruding part
- 102: step part
- 111: recessed part
- 112: contacting part
- 150: guide wire
- α: spiral inclination angle
- 6: variation
- θ: inclination angle
- A: main artery
- L1∼L3: length of step part
- R: kidney
- RA: renal artery
- RN: renal sympathetic nerve
- X: axial direction

## Claims

1. An ablation catheter comprising:
a tubular body extending in the axial direction with a spiral slit formed at least partially thereto, and
a heat element arranged at the distal end part of the tubular body which can be inserted in a living body to thermally influence the biological tissue,
wherein the tubular body include a portion having different spiral inclination angles which is an angle of the extending direction of the spiral of the slit to the cross section orthogonal to the axial direction, protruding parts formed to protrude from one side of the paired opposing surfaces that comprise the slit, and recessed parts formed on the other side of the opposing surfaces to allow the protruding parts to enter therein,
the protruding parts are formed with step parts in the portion where the angle of the slit to the spiral inclination angle changes locally, and the recessed parts are formed with contacting parts against which the step parts come in contact when the tubular body is twisted to the direction to unwind the spiral of the slit, and the length of the step parts in the axial direction is formed longer in the portion where the spiral inclination angle is relatively large than in the portion where the spiral inclination angle is small.

2. The ablation catheter according to claim 1, wherein a local variation of the angle of the slit to the spiral inclination angle is more than 90°.

3. The ablation catheter according to claim 1 or 2, wherein an inclination angle of the step part to the axial direction is within ±5°.

4. The ablation catheter according to one of claims 1 to 3 wherein the slit is **characterized in** having a gap with a constant width in between the slit.

5. The ablation catheter according to one of claim 1 to 4 wherein positions of the step parts provided on each of the slits which are aligned adjacent to each other in the axial direction of the tubular body by drawing a spiral are different in circumferential direction of the tubular body.

6. The ablation catheter according to one of claim 1 to 5 wherein the tubular body is formed with a portion where its spiral inclination angle changes gradually, and the length of the step parts plurally provided to the portion to the axial direction is formed to be gradually longer in the portion where the spiral inclination angle is large than in the portion where it is relatively small.

7. The ablation catheter according to one of claim 1 to 6 wherein the protruding parts are formed to protrude toward either the distal end side or the base side which is the side of the direction where the spiral inclination angle is larger.

8. The ablation catheter according to one of claim 1 to 7 wherein the width of the protruding parts decreases toward the protruding direction.

9. The ablation catheter according to one of claim 1 to 8 wherein the portion where the angle of the slit in the step parts changes locally is formed to have curvatures.

10. The ablation catheter according to one of claim 1 to 9 wherein the temperature measuring part is provided to the distal end side of the tubular body.

11. The ablation catheter according to one of claim 1 to 10 wherein a traction wire is provided which is inserted through the tubular body with its distal end part connected to the distal end part of the tubular body to be able to bend the tubular body by pulling the base part.

12. The ablation catheter according to one of claim 1 to 11 to be inserted within the renal artery to ablate the renal sympathetic nerve.
